# NEW EUROPEAN PATENT SPECIFICATION

(11) **EP 0 874 807 B2**
(45) Date of publication and mention of the opposition decision: **02.08.2006**
(45) Mention of the grant of the patent: 20.02.2002
(21) Application number: 96939832.0
(22) Date of filing: 18.11.1996
(51) Int. Cl.: C07C 249/08, C07C 231/06, C07C 235/78, C07C 251/48, C07C 251/60

(54) **PROCESS FOR THE PREPARATION OF METHOXYMINOPHENYLGLYOXYLIC ACID DERIVATIVES**
VERFAHREN ZUR HERSTELLUNG VON METHOXIMINOPHENYLGLYOXYLSÄURE-DERIVATEN
PROCEDE DE PREPARATION DE DERIVES DE L'ACIDE METHOXIMINOPHENYLGLYOXYLIQUE

(30) Priority: 29.11.1995 CH 338895; 21.02.1996 CH 45196
(43) Date of publication of application: 04.11.1998
(73) Proprietor: Bayer CropScience AG, 40789 Monheim (DE)
(72) Inventor: ASSERCQ, Jean-Marie, CH-1870 Monthey (CH); BREITSCHUH, Richard, 14109 Berlin (DE); PFAFF, Werner, CH-4334 Sisseln (CH)
(86) International application number: PCT/EP1996/005057
(87) International publication number: WO 1997/019912

(56) References cited:
- EP-A- 0 460 575
- EP-A- 0 463 488
- EP-A- 0 472 300
- EP-A- 0 535 928
- WO-A-95/34526

## Description

The present invention relates to a process for the preparation of o-chloromethylphenylmethoximinoglyoxylic acid esters of formula I 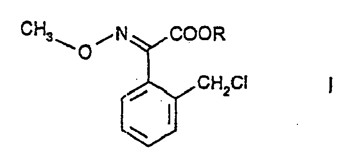 wherein R is C₃-C₈alkyl, in which process o-chloromethylphenylglyoxylic acid amide of formula III 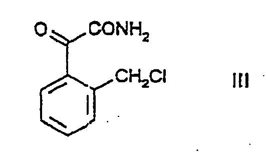 is, concurrently,
a) oximated with O-methylhydroxylamine and
b) esterified under acid conditions with a C₃-C₈alkanol by adding sulphoric acid or by introducing HCL gas into the reaction medium.

Both reaction steps are conveniently carried out in the temperature range from 20° to 70°C, preferably from 40° to 70°C. The O-methylhydroxylamine is used as such, but preferably as saft. Preferred salts are suitably those with mineral acids, such as hydrohalic acid, sulfuric acid, phosphoric acid, nitric acid and the like, but also with organic acids, such as sulfonic acids (methanesulfonic acid, ethanesulfonic acid, benzenesulfonic acid, toluenesulfonic acid, nitrobenzenesulfonic acid and the like), acetic acid, trichloroacetic acid, lactic acid, maleic acid, oxalic acid, and others.

Meanwhile a preparation method has been published (WO 95/34526) wherein a phenylacylcyanide react with an alcohol (Pinner-reaction) to the ester, which in turn can be converted with hydroxylamine and methylation or with 0-methylhydroxylamine to the oximether of formula I herein.

The compounds of formula I are valuable intermediates for obtaining E-configurated fungicides of the methoximinophenylglyoxylic acid ester series of formula IV, of which the methyl esters, in particular, are of high economic importance (EP-254426, EP-460575, EP-463488, EP-472300, EP-299694, EP-253213, WO-95/18789, WO-95/21153, and others).

The methoximinophenylglyoxylic acid ester correspond to the general formula IV 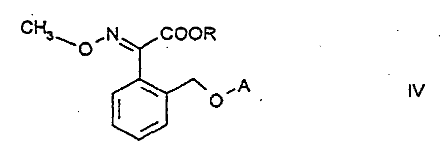 wherein
R is C₅-C₈alkyl, and
A is -N=CR₂R₃, and
R₂ = hydrogen, C₁-C₄alkyl, C₁-C₄haloalkyl, C₃-C₆cycloalkyl;
R₃=C₁-C₆alkyl, aryl-C₁-C₄alkyl, heteroaryl-C₁-C₄alkyl, C₂-C₆alkenyl, aryl-C₂-C₆alkenyl, heteroaryl-C₂-C₆alkenyl,
C₃-C₆cycloalkyl, heteroaryl, aryl, or aryl which is substituted by one to three halogen, C₁-C₄alkyl, C₁-C₄haloalkyl.
Particularly preferred compounds of formula IV are those, wherein
R₂ = hydrogen or C₁-C₄alkyl, and
R₃ = aryl, or aryl which is substituted by C₁-C₄haloalkyl.
A particularly preferred compound of formula IV is that wherein
R₂ = methyl and R₃ = 3-trifluoromethylphenyl.
A particularly preferred compound of formula IV is that, wherein R = pentyl and A= -N=C(CH₃)(3-trifiuoromethylphenyl).

Aryl is phenyl or naphthyl, preferably phenyl.

Entirely surprisingly, it has now been found that the synthesis of the methoximinoglyoxylic acid ester part of formula I and each further reaction In the chloromethyl side chain of this intermediate to a fungicide derived therefrom proceeds in particularly high purity and high yields if the methyl ester is not obtained direct from the o-chloromethylphenylglyoxylic acid amide III by methanolysis but if a long-chain ester Is prepared first and the transesterification to the methyl ester is carried out at a later stage of the process.

The o-chloromethylphenylmethoximinoglyoxylic acid esters of formula I can have the E-configuration, or a Z-configuration, at the imino double bond desirable for the biological activity.
A very special advantage of the present synthesis of compounds of formula I is that by this process, which proceeds via a long-chain ester, the E-configuration is formed almost exclusively (E/Z ratio higher than 90 : 10).

Accordingly, suitable alkanols for obtaining intermediates of formula I according to this invention are n-propanol, isopropanol, n-butanol, sec-butanol, isobutanol as well as n-pentanol, n-hexanol, n-heptanol, n-octanol and the corresponding isomers thereof. The C₅-C₆alkanols in all isomeric forms are preferred, in particular n-pentanol.

The esterification of compound III or of the corresponding oxime can be carried out in conventional manner under acid reaction conditions, e.g. by adding sulfuric acid or by introducing HCl gas into the reaction medium. The C₃-C₈alkanol serving the esterification can be used in excess and can also serve as only solvent or diluent. It can also be reacted in admixture with one or more than one inert diluent.

Suitable diluents are, for example, hydrocarbons or halogenated hydrocarbons, typically hexane, heptane, octane, chloroform, dichloroethane; aromatic hydrocarbons or aromatic halogenated hydrocarbons, typically toluene, xylene, chlorobenzene, dichlorobenzene; or ethers such as dioxane, tetrahydrofuran, diisopropyl ether; ketones such as acetone, methyl isobutyl ketone, cyclopentanone; or mixtures thereof.

The esterification can be carried out either direct with exclusion of water or under aqueous conditions via prior hydrolysis.

The hydrolysis of the amide II is preferaby carried out in the presence of stoichiometric amounts of water in the form of the corresponding aqueous concentrated hydrohalic acid, preferably In hydrochloric acid, at a concentration of 25% or more.

The alkanolysis can be catalysed by small amounts of sulfuric acid or phosphoric acid. Said alkanolysis is carried out in the temperature range from -10° to +120°C, preferably from 0° to 50°C and, particularly preferably, from 20° to 50°C in the presence of the C₃-C₈alkanol.

Accordingly, hydrolysis and esterification proceed in one single reaction step of the alkanolysis.

The reaction of the keto group in the compound of formula III with O-methylhydroxylamine or its salt can be carried out before or after this alkanolysis step of compound III, but is preferably carried out concurrently. It is an essential feature of this invention that the conversion of III to I can be carried out in one single process step.

The compounds of formula I are useful as intermediates containing a reactive o-chloromethyl group for preparing pesticides. Such pesticides can be fungicides, insecticides, acaricides, nematicides or herbicides such as are disclosed in EP-A-254426, EP-A-460575, EP-A-463488, EP-A-472300, EP-A-299694, EP-A-253213, WO-95/18799, WO-95/21153 as well as in other publications.

### Working Examples

### W-1 : Preparation of o-chloromethylpheny glyoxylic acid amide

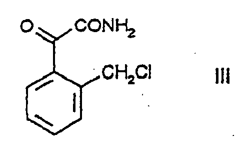

A) 20 g of hydrochloric gas (0.55 mol) are Introduced to a suspension of 179.6 g of o-chloromethylbenzoyl cyanide (1 mol) in 400 g of acetic acid over 1 hour. To this mixture are then added 31.8 g of 32% aqueous hydrochloric acid (1.2 mol of water). The suspension is stirred for a further 28 hours at room temperature. The product, which is partly crystallised, is isolated by filtration, washed with 2x50 mi of n-pentanol and 2x50 ml of hexane and then dried. Yeld: 89.2 g (45.1 %). The mother liquor is recycled in the next batch as diluent.
B) 33.1 g of acetic acid are added to a suspension of 179.6 g of o-chloromethylbenzoyl cyanide (1 mol) in 482.9 g of mother liquor of the preceeding batch and then 5 g of hydrochloric gas (0.14 mol) are introduced. 24.3 g of aqueous 32% hydrochloric acid (0.92 mol of water) are added to the reaction mixture at room temperature. The reaction mixture is stirred for 21 hours at room temperature and then filtered. The crystals are washed with pentanol and hexane and then dried. Yield: 172.8 g (87.4%). The mother liquor (486.1 g) is used as diluent in the next batch.

### W-2 : Preparation of o-chloromethylphenyl-O-methoximinoglyoxylic acid pentyl ester

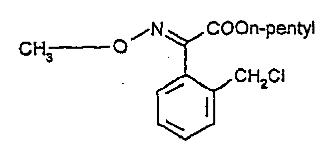

Over one hour, 50 g (1.37 mol) of hydrochloric acid gas are introduced to a cooled suspension (0° to -10°C) of 41.8 g of O-methylhydroxylamine hydrochloride (0.5 mol) in 250 g of n-pentanol. The mixture is then heated to about 50°-55°C and a first portion of o-chloromethylphenylglyoxylic acid amide (49.4 g; 0.25 mol) is added. The mixture is stirred for 2 hours at 50°-55°C and then a second portion of o-chloromethylphenylglyoxylic acid amide (49.4 g; 0.25 mol) is added. The mixture is stirred for 24 hours at 50-60°C and then cooled to 20°C amd diluted with 175 ml of water. 159.7 g of a 30% aqueous sodium hydroxide solution are added such that the temperature is kept at 20°-30°C. The organic phase is isolated, dried and concentrated. Yield: 133.1 g (79%).
The E/Z isomer ratio based on the O-methyloxime side chain is 92.6: 7.4.
Replacing n-pentanol in the above process with methanol would result in a very unfavourable E/Z isomer ratio of 83 :17,

### W-3 : Preparation of 2-[α-{[(α-methyl-3-trilluoromethylbenzyl)imino]oxy}-o-tolyl]glyoxylic acid pentyl ester-O-methyloxime

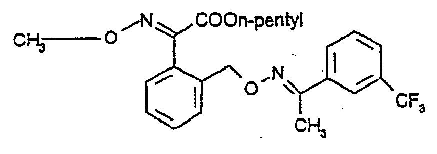

87 g of a 30% sodium methylate solution in methanol are added dropwise to a solution of 98.2 g of 3-trifluoromethylacetophenonoxime (0.48 mol) in 200 ml of dimethyl acetamide over 20 minutes. 196 ml of solvent are then distilled off under a slight vacuum at 55°-70°C. After the addition of 1 g of potassium iodide, 150 g of o-chloromethylphenyl-O-methoximinoglyoxylic acid pentyl ester having a purity of 95.9% (0.48 mol) are added over 1 hour at 55°-65°C. After2 hours, the reaction mixture is added over 30 minutes to a mixture of 400 ml of water and 250 ml of toluene at room temperature, the pH being adjusted to 5 with concentrated hydrochloric acid. The aqueous phase is extracted twice with 150 ml of toluene. The combined organic phases are washed with 150 mi of water. The solvent is distilled off under vacuum at 60°C. 227 g of crude oil are obtained having a 83% content.

### W-3 : Preparation of 2-[α-{[(α-methyl-3-trifluoromethylbenzyl)imino]oxy}-o-tolyl]glyoxylic acid pentyl ester-0-methyloxime

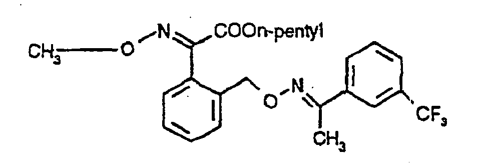

87 g of a 30% sodium methylate solution in methanol are added dropwise to a solution of 98.2 g of 3-trifluoromethylacetophenonoxime (0.48 mol) in 200 ml of dimethyl acetamide over 20 minutes. 196 ml of solvent are then distilled off under a slight vacuum at 55°-70°C. After the addition of 1 g of potassium iodide, 150 g of o-chloromethylphenyl-O-methoximinoglyoxylic acid pentyl ester having a purity of 95,9% (0.48 mol) are added over 1 hour at 55°-65°C. After 2 hours, the reaction mixture is added over 30 minutes to a mixture of 400 ml of water and 250 ml of toluene at room temperature, the pH being adjusted to 5 with concentrated hydrochloric acid. The aqueous phase is extracted twice with 150 ml of toluene. The combined organic phases are washed with 150 ml of water. The solvent is distilled off under vacuum at 60°C. 227 g of crude oil are obtained having a 83% content.

## Claims

1. A process for the preparation of an o-chloroinethylphenylinethoximinoglyoxylic acid ester of formula (I), 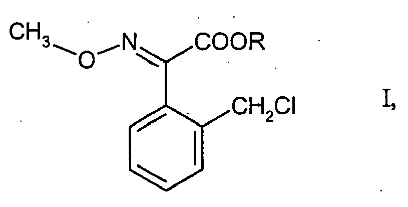 wherein R is C₃-C₈-akyl, in which process o-chloromethylphenylglyoxylic acid amide of formula III 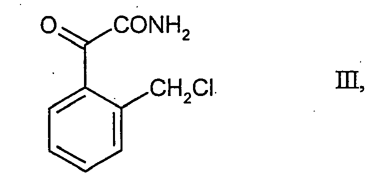 is concurrently
a) oximated with O-methylhydroxylamine and
b) esterified under acid conditions with a C₃-C₈-alkanol by adding sulphuric acid or by introducing HCl gas into the reaction medium
in the presence or absence of a diluent.

2. A process according claim 1, which comprises carrying out the reaction in the temperature range from 20°C to 70°C.

3. A process according to claim 2, which comprises carrying out the reaction in the temperature range from 40°C to 70°C.

4. A process according to one or more of claims 1 to 3 which comprises using the C₃-C₈-alkanol reactant as diluent.

5. A process according to claim 4, which comprises using n-pentanol.

## Patentansprüche

1. Verfahren zur Herstellung eines o-Chlormethylphenylmethoximinoglyoxylsäureesters der Formel (I): 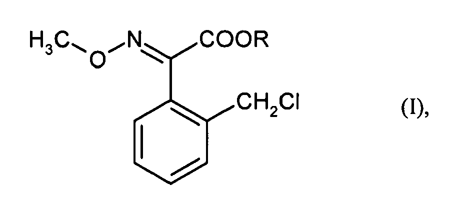 worin R ein C₃₋₈-Alkylrest ist, in welchem Verfahren o-Chlormethylphenylglyoxylsäureamid der Formel (III): 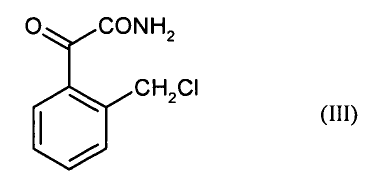 gleichzeitig
a) mit O-Methylhydroxylamin in das Oxim überführt und
b) unter sauren Bedingungen mit einem C₃₋₈-Alkanol, in der Gegenwart oder Abwesenheit eines Verdünnungsmittels verestert wird, wobei Schwefelsäure oder Chlorwasserstoffgas als Säure verwendet werden.

2. Verfahren gemäß Anspruch 1, wobei man die Reaktion im Temperaturbereich von 20 bis 70°C durchführt.

3. Verfahren gemäß Anspruch 2, wobei man die Reaktion im Temperaturbereich von 40 bis 70°C durchführt.

4. Verfahren gemäß einem oder mehreren der Ansprüche 1 - 3, wobei man den C₃₋₈-Alkanolreaktionsteilnehmer als Verdünnungsmittel verwendet.

5. Verfahren gemäß Anspruch 4, wobei man n-Pentanol verwendet.

## Revendications

1. Procédé pour la préparation d'un ester d'acide o-chlorométhylphénylméthoximinoglyoxylique de formule I: 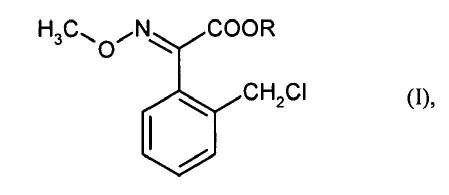 dans laquelle R représente un groupe alkyle en C₃ à C₈, procédé dans lequel de l'amide d'acide o-chlorométhylphénylglyoxylique de formule III: 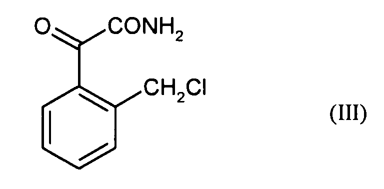 est conjointement
a) oximé avec de la O-méthylhydroxylamine et
b) esterifie dans les conditions acides en introduissant l'acide sulfurique ou gaz de chlorure d'hydrogène avec un alcanol en C₃ à C₈, en la présence ou en l'absence d'un diluant.

2. Procédé suivant la revendication 1, qui comprend la conduite de la réaction dans la plage de températures de 20°C à 70°C.

3. Procédé suivant la revendication 2, qui comprend la conduite de la réaction dans la plage de températures de 40°C à 70°C.

4. Procédé suivant un ou plusieurs revendications 1 à 3, qui comprend l'utilisation du corps réactionnel consistant en un alcanol en C₃ à C₈ comme diluant.

5. Procédé suivant la revendication 4, qui comprend l'utilisation de n-pentanol.
